# EUROPEAN PATENT APPLICATION

(11) **EP 4 389 147 A2**
(43) Date of publication of application: **26.06.2024**
(21) Application number: 24174213.9
(22) Date of filing: 09.08.2018
(51) Int. Cl.: A61K 47/10

(54) **THERAPEUTIC FORMULATIONS AND USES THEREOF**

(30) Priority: 09.08.2017 US 201762543215 P; 04.12.2017 US 201762594399 P
(62) Divisional of application: 18843332.0
(71) Applicant: Dechra Veterinary Products, LLC, Kansas 66211 (US)
(72) Inventor: HEPLER, Douglas, North Carolina, 27410 (US); DEMPSEY, Gail L., North Carolina, 27410 (US); JOHNSON, Roland, North Carolina, US, 27410 (US); KELLY, Michael, North Carolina, 27410 (US); DANIEL, Michael, North Carolina, 27410 (US); PAULSEN, Neil, E., North Carolina, 27410 (US); CLAYTON, Bert, North Carolina, US, 27410 (US)
(74) Representative: Hadfield, Andrea

(57) **Abstract**

Provided herein are pharmaceutically acceptable compositions containing a selective cyclooxygenase-2 (COX-2) inhibitor (coxib) and optionally buprenorphine. In particular, compositions containing a coxib formulated for oral, topical or subcutaneous administration to treat pain or inflammation are described.

## Description

### CROSS-REFERENCE TO RELATED APPLICATIONS

This application claims benefit of priority under 35 U.S.C. §119(e) of U.S. Serial No. 62/543,215, filed August 9, 2017, and U. S. Serial No. 62/594,399, filed December 4, 2017, the entire contents of both are incorporated herein by reference in its entirety.

### BACKGROUND OF THE INVENTION

### FIELD OF THE INVENTION

The invention relates generally to therapeutic formulations and more specifically to formulations including a selective cyclooxygenase-2 (COX-2) inhibitor (coxib) and optionally buprenorphine.

### BACKGROUND INFORMATION

Nonsteroidal anti-inflammatory drugs (NSAIDs) inhibit both COX-1 and COX-2 enzymes and have been commonly used in veterinary medicine for the treatment of inflammation and pain, (COX is the commonly accepted abbreviation for cyclooxygenase the enzyme that produces prostaglandins from arachidonic acid). While the specificity for which COX isoform a given NSAID inhibits varies depending on the details of the particular assay employed and their chemical structure, as a class all NSAIDs inhibit both COX-1 and COX-2 enzymes. Among the numerous chemotypes of NSAID drugs, the propionic acid derivatives are the most common.

COX-2-selective inhibitors (coxibs) developed in the 2000s supplanted traditional NSAIDs in some settings by virtue of their comparable efficacy with improved gastrointestinal safety. Several COX-2 inhibitors are available for treatment of pain and inflammation in companion animals. However, it is particularly desirable to have a coxib formulation which can be safely provided with long-lasting results.

### SUMMARY OF THE INVENTION

The present invention is based on the seminal discovery of compositions containing a coxib and optionally buprenorphine to provide prolonged treatment of pain and inflammatory disorders in mammals.

As such, provided herein are compositions containing a coxib and optionally buprenorphine.

In one aspect, the composition is formulated as an injectable including:
(a) at least about 0.5% w/w and up to 50% w/w (and all concentrations in between) of a coxib; and
(b) a solvent, wherein the composition is formulated for subcutaneous administration.

In embodiments the injectable composition includes:
(c) a solvent optionally including at least 0.1% and up to about 85% of one or more excipients (e.g., polyethylene glycol, "PEG" and/or propylene glycol);
(d) optionally up to 25% w/w of at least one optional excipient (e.g., ethanol), preservative, penetration enhancer and/or viscosity reducer; and
(e) optionally up to 10% w/w of at least one anti-oxidant.

In another aspect, the composition is formulated in a topical dosage form including:
(a) at least about 0.5% w/w and up to 50% w/w (and all concentrations in between) of a coxib; and
(b) a solvent, wherein the composition is formulated for topical administration.

In embodiments the topical composition includes:
(c) a solvent optionally including at least 1.0% and up to about 99.5% of one or more excipients (e.g., propylene glycol);
(d) optionally up to 75% w/w of at least one penetration enhancer (e.g., propylene carbonate and/or n-methyl pyrrolidone);
(e) optionally up to 25% w/w of at least one optional solvent and/or penetration enhancer (e.g., ethanol and/or n-methyl pyrrolidone); and
(f) optionally up to 10% w/w of at least one anti-oxidant.

In another aspect, the composition is formulated in an oral dosage form including:
(a) at least about 0.5% w/w and up to 90% w/w (and all concentrations in between) of a coxib;
(b) optionally up to about 99.5% of a suitable pharmaceutical carrier (e.g., lactose monohydrate and/or microcrystalline cellulose); and
(c) optionally flavoring(s), binder(s), disintegrate(s), lubricant(s) and/or glidant(s).

In various embodiments, the formulations of the invention (e.g., oral, injectable and topical) include one or more coxibs, such as mavacoxib, or a chromene coxib (as described further herein), such as PAH-100 as shown in Formula (I) or Formula (II): 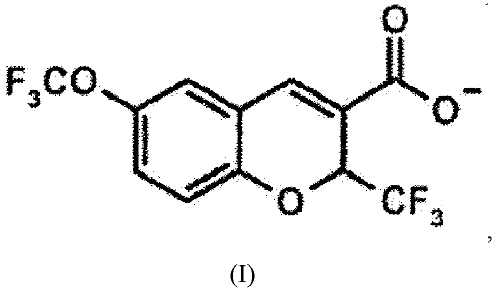 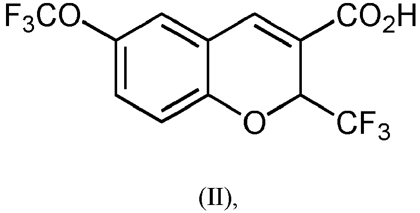 or isomers or pharmaceutically acceptable salts thereof.

In some embodiments, the formulations include a coxib and optionally buprenorphine and/or one or more NSAIDS.

Also provided herein is a method of treating a disease or disorder (e.g., pain or an inflammatory disorder) in a subject by administering a formulation of the invention. Surprisingly, a clinically effective amount of the coxib when provided in a formulation of the invention is present in the blood stream of the subject for at least 96, 120, 144 or 168 hours after administration. In various embodiments, buprenorphine is administered along with the coxib, either in a single formulation or in a separate formulation that is co-administered along with the coxib. In a particularly preferred embodiment, a formulation of the invention is delivered to a companion animal such as a cat or dog.

### BRIEF DESCRIPTION OF THE FIGURES

Figure 1 is a graph showing pharmacokinetic (PK) curves for oral, subcutaneous injectable and topical formulations of the disclosure administered to a mammal via their respective routes of administration in embodiments of the invention.
Figure 2 is a graph showing plasma concentrations for formulations of the disclosure administered to subjects in embodiments of the invention.
Figure 3 is a graph showing plasma concentrations for formulations of the disclosure administered to subjects in embodiments of the invention.
Figure 4 is a graph showing plasma concentrations for formulations of the disclosure administered to subjects in embodiments of the invention.
Figure 5 is a graph showing plasma concentrations for formulations of the disclosure administered to subjects in embodiments of the invention.
Figure 6 is a graph showing plasma concentrations for formulations of the disclosure administered to subjects in embodiments of the invention.

### DETAILED DESCRIPTION OF THE INVENTION

The following terms, definitions and abbreviations apply. Abbreviations used herein have their conventional meaning within the chemical and biological arts.

As used herein, a "patient" or "subject" refers to either a human or non-human mammalian animal. Non-human animals include any non-human mammalian animals. Such non-human animals may include, but are not limited to rodents, non-human primates (e.g., monkey and apes), ungulates, ovines, bovines, ruminants, lagomorphs, porcines, caprines, equines, canines, felines, murines, and the like. In certain embodiments of the invention, the animals are mammals. In the context of the disclosure, the term "subject" generally refers to an individual who will receive or who has received treatment described below (e.g., administration of a composition of the disclosure, and optionally one or more additional therapeutic agents).

The term "therapeutically effective amount" means the amount of the compound or pharmaceutical composition that will elicit the biological or medical response of a patient or tissue that is being sought by the researcher, veterinarian, medical doctor or other clinician.

By "pharmaceutically acceptable" it is meant the carrier, diluent or excipient must be compatible with the other ingredients of the formulation and not deleterious to the recipient thereof.

The terms "administration of" and or "administering a" compound should be understood to mean providing a compound of the disclosure or pharmaceutical composition to the subject in need of treatment.

The disclosure provides pharmaceutical compositions comprising at least one coxib in an amount effective for treating a disorder, such as pain or inflammation, and a pharmaceutically acceptable vehicle or diluent. As used herein the term "coxib" refers to a selective cyclooxygenase-2 (COX-2) inhibitor or "COX-2 inhibitor".

In embodiments, the coxib may be any coxib known in the art, for example, an in now was limiting, mavacoxib, rofecoxib, celecoxib, cimicoxib, deracoxib, firocoxib, robenacoxib, valdecoxib, parecoxib, etoricoxib or any combination thereof.

In some embodiments, the coxib is mavacoxib, a non-steroidal anti-inflammatory drug (NSAID) of the coxib class (ATCvet Code QM01AH92). Mavacoxib - 4-[5-(4-fluorophenyl)-3-(trifluoromethyl)-1H-pyrazol-1-yl]benzenesulfonamide is used in the treatment of chronic pain and inflammation associated with osteoarthritis in canines. It is a specific inhibitor of the inducible form of cyclooxygenase.

In some embodiments, the coxib is a "chromene coxib" and is referred to herein as PAH-100. The coxib PAH-100 is a member of a structural class of COX-2 selective inhibitors. As used herein, PAH-100 refers of a compound of Formula (I): 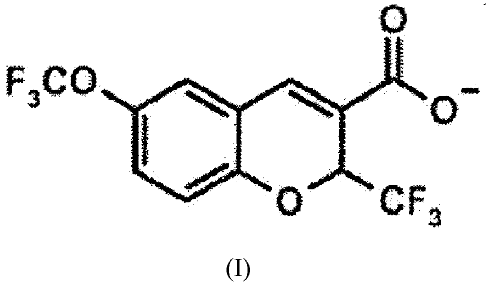 or an isomer or pharmaceutically acceptable salt thereof, or a compound of Formula (II): 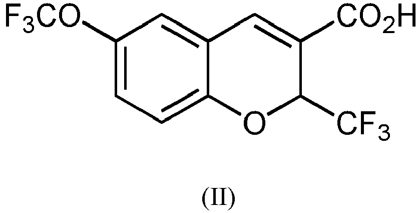 or an isomer or pharmaceutically acceptable salt thereof.

Particular isomers or pharmaceutically acceptable salts of PAH-100 for use in compositions of the invention include Tris(hydroxymethyl)aminomethane (±)-6-(trifluoromethoxy)-2-(trifluoromethyl)-2H-chromene-3-carboxylate, (±)-6-(trifluoromethoxy)-2-(trifluoromethyl)-2H-chromene-3-carboxylic acid, Tris(hydroxymethyl)aminomethane (R)-6-(trifluoromethoxy)-2-(trifluoromethyl)-2H-chromene-3-carboxylate, (R)-6-(trifluoromethoxy)-2-(trifluoromethyl)-2H-chromene-3-carboxylic acid, Tris(hydroxymethyl)aminomethane (S)-6-(trifluoromethoxy)-2-(trifluoromethyl)-2H-chromene-3-carboxylate, and (S)-6-(trifluoromethoxy)-2-(trifluoromethyl)-2H-chromene-3-carboxylic acid.

In embodiments, PAH-100 may be a racemic mixture, for example a mixture of Tris(hydroxymethyl)aminomethane (R)-6-(trifluoromethoxy)-2-(trifluoromethyl)-2H-chromene-3-carboxylate and Tris(hydroxymethyl)aminomethane (S)-6-(trifluoromethoxy)-2-(trifluoromethyl)-2H-chromene-3-carboxylate, or a mixture of (R)-6-(trifluoromethoxy)-2-(trifluoromethyl)-2H-chromene-3-carboxylic acid and (S)-6-(trifluoromethoxy)-2-(trifluoromethyl)-2H-chromene-3-carboxylic acid.

A key structural feature of PAH-100 is the presence of a benzopyran (also known as chromene) moiety having the following structure:

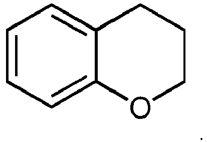

Chromene coxibs are selective COX-2 inhibitors based on this very unique central chemical ring system. Unlike NSAIDs and other COX-2 inhibitors, PAH-100 does not possess a propionic acid moiety or a sulfone or sulfonamide group. The presence of a carboxylic acid endows PAH-100 with an unexpected and advantageous degree of aqueous solubility allowing for rapid oral absorption. In addition, PAH-100 has a relatively long duration of action *in vivo* making for a more convenient dosing regimen. The inventors have also discovered that mavacoxib, while being of a different structural claim than PAH-100 exhibits pharmacokinetic characteristics comparable to PAH-100. As such, both compounds are uniquely suitable for use in the compositions of the invention.

The compositions of the disclosure may contain therapeutic agents in addition to a coxib and may be formulated, for example, by employing solid or liquid vehicles or diluents, as well as pharmaceutical additives of a type appropriate to the mode of desired administration (for example, excipients, binders, preservatives, stabilizers, flavors, etc.) according to techniques such as those well known in the art of pharmaceutical formulation.

Additional excipients which are contemplated for use in the practice of the disclosure are those available to those of ordinary skill in the art, for example, those found in the United States Pharmacopeia Vol. XXII and National Formulary Vol. XVII, U.S. Pharmacopeia Convention, Inc., Rockville, Md. (1989) as well as updates thereto, such as United States Pharmacopeia Vol. XL and National Formulary Vol. XXXV, U.S. Pharmacopeia Convention, Inc., Rockville, Md., the relevant contents of which are incorporated herein by reference. In addition, polymorphs, hydrates, and solvates of the compounds are included in the disclosure, with hydrates being particularly preferred. It should be noted that while the hydrate molecules will contribute water to the pharmaceutical composition, it is most preferred that no other water source be included.

Also, while the coxibs may be in their hydrated form, no water is added to the composition during or after mixture. As such, the compositions described herein are substantially non-aqueous, for example, the compositions have less than about 6.0, 5.5, 5.0, 4.5, 4.0, 3.5, 3.0, 2.5, 2.0, 1.5, 1.0, 0.9, 0.8, 0.7, 0.6, 0.5, 0.4, 0.3, 0.2, 0.1, 0.5 or 0.1% w/w of an aqueous substance, such as water.

The disclosed pharmaceutical compositions could be administered by any suitable means, for example, orally, sublingually; buccally; parenterally, such as by subcutaneous, intravenous, intramuscular, intrathecal, or intracisternal injection or infusion techniques (e.g., as sterile injectable non-aqueous solutions or suspensions); nasally such as by inhalation spray; topically, such as in the form of a cream or ointment; or rectally such as in the form of suppositories; in dosage unit formulations containing non-toxic, pharmaceutically acceptable vehicles or diluents. Preferably, however, the administration will be by injection, topically or orally.

The pharmaceutical compositions of the present invention are for the administration of a coxib either alone or in combination with other agents, for example, anti-inflammatories, analgesics, antibiotics, anti-fungals, anti-virals and other pharmaceutically active components, although the compositions are therapeutically effective when a coxib is the sole active agent present.

In embodiments, the compositions may include coxib and an analgesic, such as buprenorphine. Buprenorphine is a synthetic opioid drug that is about 30 times more potent than morphine..

In embodiments, the compositions may include include a coxib and one or more additional NSAIDS. As used herein, the term "NSAID" refers to a class of therapeutic compounds with analgesic, anti-inflammatory, and anti-pyretic properties. NSAIDs reduce inflammation by blocking cyclooxygenase. NSAIDs may be classified based on their chemical structure or mechanism of action. Non-limiting examples of NSAIDs include a salicylate derivative NSAID, a p-amino phenol derivative NSAID, a propionic acid derivative NSAID, an acetic acid derivative NSAID, an enolic acid derivative NSAID, a fenamic acid derivative NSAID, a non-selective cyclooxygenase (COX) inhibitor, a selective cyclooxygenase 1 (COX-1) inhibitor, and a selective cyclooxygenase 2 (COX-2) inhibitor. A NSAID may be a profen. Examples of a suitable salicylate derivative NSAID include, without limitation, Acetylsalicylic acid (asprin), Diflunisal, Hydroxylethyl Salicylate, and Salsalate. Examples of a suitable p-amino phenol derivative NSAID include, without limitation, Paracetamol and Phenacetin. Examples of a suitable propionic acid derivative NSAID include, without limitation, Alminoprofen, Benoxaprofen, Dexketoprofen, Fenoprofen, Flurbiprofen, Ibuprofen, Indoprofen, Ketoprofen, Loxoprofen, Naproxen, Oxaprozin, Pranoprofen, And Suprofen. Examples of a suitable acetic acid derivative NSAID include, without limitation, Aceclofenac, Acemetacin, Actarit, Alcofenac, Aloxipirin, Amfenac, Aminophenazone, Antraphenine, Azapropazone, Benorilate, Benzydamine, Butibufen, Chlorthenoxacine, Choline Salicylate, Clometacin, Diclofenac, Emorfazone, Epirizole, Etodolac, Feclobuzone, Felbinac, Fenbufen, Fenclofenac, Glafenine, Indometacin, Ketorolac, Lactyl Phenetidin, Metamizole, Metiazinic Acid, Mofebutazone, Mofezolac, Nabumetone, Nifenazone, Niflumic Acid, Oxametacin, Pipebuzone, Propyphenazone, Proquazone, Protozininc Acid, Salicylamide, Sulindac, Tiaramide, Tinoridine, and Zomepirac. Examples of a suitable enolic acid (Oxicam) derivative NSAID include, without limitation, Droxicam, Isoxicam, Lomoxicam, Meloxicam, Piroxicam, and Tenoxicam. Examples of a suitable fenamic acid derivative NSAID include, without limitation, Flufenamic acid, Mefenamic acid, Meclofenamic acid, and Tolfenamic acid. Examples of a suitable selective COX-2 inhibitor include, without limitation, Celecoxib, Etoricoxib, Firocoxib, Lumiracoxib, Meloxicam, Parecoxib, Rofecoxib, and Valdecoxib.

Moreover, the compositions may include analgesic agents other than anti-inflammatory agents, such as opiates, local anesthetics such as Lidocain, Mepivacain, Prilocain, Procain, Syntocain, Tetracain, Gingicain, Articain, Bupivacain, Butanilicain, Chloroprocain, or, for example, Polidocanol.

Furthermore, the compositions may also include anti-inflammatory agents that could have a secondary effect as analgesics other than the analgesics listed above, which may in part have anti-inflammatory effects, such as hormones, specifically Cortison and corticoids, such as glucocorticoids (e.g., Cortison, Cloprednol, Prednison, Prednisolon, Methylprednisolon, Deflazacort, Fluocortolon, Triamcinolon, Dexamethason, Betamethason) and mineralcorticoids (e.g. Aldosteron, Desoxycorticosteron, Fludrocortison).

The composition may conveniently be presented in dosage unit form and may be prepared by any of the methods well known in the art of pharmacy, for example those methods described in the Examples. All methods include the step of bringing the active ingredient into association with the carrier which constitutes one or more accessory ingredients. In general, the pharmaceutical compositions are prepared by uniformly and intimately bringing the active ingredient into association with a carrier suitable for administration via an intended route, for example, injection, oral or topical administration routes. In the pharmaceutical composition, the active object compound is included in an amount sufficient to produce the desired effect upon the process or condition of diseases.

Exemplary topical formulations of the invention include:
(a) a coxib, such as PAH-100 or mavacoxib; and
(b) a solvent, wherein the composition is formulated as a topical dosage form.

In embodiments, the solvent includes propylene glycol present at over about 1% w/w of the composition.

In embodiments the composition optionally includes an anti-oxidant present at no more than 10% w/w of the composition,.

Particular embodiments of formulations suitable for topical use are as follows.

| **Topical Formulation** | | | | |
|---|---|---|---|---|
| **Ingredient** | **Ideal w/w %** | **Range w-w%** | **Purpose** | **Exemplary Alternates** |
| Mavacoxib | 5 | 0.5-50 | API | any other coxib or combination thereof |
| Propylene Glycol (PG) | 40 | 1-99.5 | Solvent | n-methyl pyrrolidone (NMP), PEG300, PEG400, water, 2-pyrrolidone, dimethyl sulfoxide (DMSO), Triacetin, Triethylcitrate (TEC), glycerin |
| Propylene Carbonate (PC) | 40 | 0-75 | Penetration Enhancer | Triacetin, Triethylcitrate (TEC), benzyl alcohol, n-methyl pyrrolidone (NMP) |
| Ethanol | 15 | 0-25 | Co-solvent, additional penetration enhancer, preservative, viscosity reducer | Isopropanol (IPA), benzyl alcohol, capryllic triglycerides, triolein, n-methyl pyrrolidone (NMP) |
| Optional | 0 | 0.002-10 | anti-oxidants | anti-oxidants (propyl gallate, BHT, BHA, Tenox 4 and other Tenoxes, Vitamin E and analogs, TBHQ, methylparaben, propylparaben, and all other anti-oxidants) |
| Total | 100 | | | |

Particular embodiments of formulations suitable for topical use are as follows.

| **Topical Formulation** | | | | |
|---|---|---|---|---|
| **Ingredient** | **Ideal w/w** % | **Range w-w%** | **Purpose** | **Exemplary Alternates** |
| PAH-100 | 5 | 0.5-50 | API | any other coxib or combination thereof |
| Propylene Glycol (PG) | 40 | 1-99.5 | Solvent | n-methyl pyrrolidone (NMP), PEG300, PEG400, water, 2-pyrrolidone, dimethyl sulfoxide (DMSO), Triacetin, Triethylcitrate (TEC), glycerin |
| Propylene Carbonate (PC) | 40 | 0-75 | Penetration Enhancer | Triacetin, Triethylcitrate (TEC), benzyl alcohol, n-methyl pyrrolidone (NMP) |
| Ethanol | 15 | 0-25 | Co-solvent, additional penetration enhancer, preservative, viscosity reducer | Isopropanol (IPA), benzyl alcohol, capryllic triglycerides, triolein, n-methyl pyrrolidone (NMP) |
| Optional | 0 | 0.002-10 | anti-oxidants | anti-oxidants (propyl gallate, BHT, BHA, Tenox 4 and other Tenoxes, Vitamin E and analogs, TBHQ, methylparaben, propylparaben, and all other anti-oxidants) |
| Total | 100 | | | |

In one embodiment, an exemplary topical formulation is as follows:

| **Topical Formulation** | | |
|---|---|---|
| **Master Formula** | **w/w%** | **ID: PAH-16-04-004** |
| Mavacoxib | 7.5 | |
| Propylene Glycol (PG) | 45 | |
| Propylene Carbonate (PC) | 27.5 | |
| n-methyl pyrrolidone (NMP) | 20 | |
| **Total** | 100 | |

In one embodiment, an exemplary topical formulation is as follows:

| **Topical Formulation** | |
|---|---|
| **Master Formula** | **w/w%** |
| PAH-100 | 7.5 |
| Propylene Glycol (PG) | 45 |
| Propylene Carbonate (PC) | 27.5 |
| n-methyl pyrrolidone (NMP) | 20 |
| **Total** | 100 |

In one embodiment, an exemplary topical formulation is as follows:

| **Topical Formulation** | |
|---|---|
| **Master Formula** | **w/w%** |
| PAH-100 | 5 |
| Propylene Glycol (PG) | 40 |
| Propylene Carbonate (PC) | 40 |
| Ethanol | 15 |
| **Total** | 100 |

In one embodiment, an exemplary topical formulation is as follows:

| **Topical Formulation** | |
|---|---|
| **Master Formula** | **w/w%** |
| Mavacoxib | 5 |
| Propylene Glycol (PG) | 40 |
| Propylene Carbonate (PC) | 40 |
| Ethanol | 15 |
| **Total** | 100 |

In one embodiment, a topical composition includes: i) a coxib; ii) propylene glycol; iii) propylene carbonate; iv) ethanol; and v) optionally an anti-oxidant.

In one embodiment, a topical composition includes: i) a coxib at a concentration of about 0.5 to 50% w/w; propylene glycol at a concentration of about 1 to 99% w/w; iii) propylene carbonate at a concentration of about 0.001 to 75% w/w; and iv) ethanol at a concentration of about 0.001 to 25% w/w.

In one embodiment, a topical composition includes: i) coxib at a concentration of about 5 to 15% w/w; ii) propylene glycol at a concentration of about 35 to 45% w/w; iii) propylene carbonate at a concentration of about 35 to 45% w/w; and iv) ethanol at a concentration of about 5 to 20% w/w.

Exemplary injectable formulations of the invention include:
(a) a coxib, such as PAH-100 or mavacoxib; and
(b) a solvent, wherein the composition is formulated for subcutaneous administration.

In embodiments the injectable composition includes:
(c) a solvent optionally including at least 0.1% and up to about 85% of one or more excipients (e.g., polyethylene glycol, "PEG" and/or propylene glycol);
(d) optionally up to 25% w/w of at least one optional excipient (e.g., ethanol), preservative, penetration enhancer and/or viscosity reducer; and
(e) optionally up to 10% w/w of at least one anti-oxidant.

Particular embodiments of formulations suitable for subcutaneous administration are as follows.

| **Injectable Formulation** | | | | |
|---|---|---|---|---|
| **Ingredient** | **Ideal w/w %** | **Range w/w%** | **Purpose** | **Exemplary Alternatives** |
| Mavacoxib | 5 | 0.5-50 | API | any other coxib or combination thereof |
| Propylene Glycol | 50 | 1-60 | Solvent/Co-solvent | n-methyl pyrrolidone (NMP), PEG300, PEG400, water, 2-pyrrolidone, dimethyl sulfoxide (DMSO), Triacetin, Triethylcitrate (TEC), glycerin |
| Polyethylene glycol 300 (PEG300) | 40 | 0.5-85 | Solvent/Co-solvent | n-methyl pyrrolidone (NMP), Propylene glycol, PEG400, water, 2-pyrrolidone, dimethyl sulfoxide (DMSO), Triacetin, Triethylcitrate (TEC), medium chain triglycerides |
| Ethanol | 5 | 0-25 | Co-solvent, additional penetration enhancer, preservative, viscosity reducer | Isopropanol (IPA), benzyl alcohol, capryllic triglycerides, triolein |
| Other | 0 | 0.002-10 | anti-oxidants | anti-oxidants (propyl gallate, BHT, BHA, Tenox 4 and other Tenoxes, Vitamin E and analogs, TBHQ, methylparaben, propylparaben, and all other anti-oxidants) |
| Total | 100 | | | |

Particular embodiments of formulations suitable for subcutaneous administration are as follows.

| **Injectable Formulation** | | | | |
|---|---|---|---|---|
| **Ingredient** | **Ideal w/w %** | **Range w/w%** | **Purpose** | **Exemplary Alternatives** |
| PAH-100 | 12 | 0.5-50 | API | any other coxib or combination thereof |
| Propylene Glycol | 50 | 1-60 | Solvent/Co-solvent | n-methyl pyrrolidone (NMP), PEG300, PEG400, water, 2-pyrrolidone, dimethyl sulfoxide (DMSO), Triacetin, Triethylcitrate (TEC), glycerin |
| Polyethylene glycol 300 (PEG300) | 33 | 0.5-85 | Solvent/Co-solvent | n-methyl pyrrolidone (NMP), Propylene glycol, PEG400, water, 2-pyrrolidone, dimethyl sulfoxide (DMSO), Triacetin, Triethylcitrate (TEC), medium chain triglycerides |
| Ethanol | 5 | 0-25 | Co-solvent, additional penetration enhancer, preservative, viscosity reducer | Isopropanol (IPA), benzyl alcohol, capryllic triglycerides, triolein |
| Other | 0 | 0.002-10 | anti-oxidants | anti-oxidants (propyl gallate, BHT, BHA, Tenox 4 and other Tenoxes, Vitamin E and analogs, TBHQ, methylparaben, propylparaben, and all other anti-oxidants) |
| Total | 100 | | | |

In one embodiment, a topical composition includes: i) a coxib; ii) propylene glycol; iii) polyethylene glycol; iv) ethanol; and optionally v) an anti-oxidant.

In one embodiment, a topical composition includes: i) a coxib at a concentration of about 0.5 to 50% w/w; ii) propylene glycol at a concentration of about 1 to 60% w/w; iii) polyethylene glycol at a concentration of about 0.5 to 85% w/w; and iv) ethanol at a concentration of about 0.001 to 25% w/w.

In one embodiment, a topical composition includes: i) a coxib at a concentration of about 5 to 15% w/w; ii) propylene glycol at a concentration of about 45 to 55% w/w; iii) polyethylene glycol at a concentration of about 30 to 35% w/w; and iv) ethanol at a concentration of about 1 to 10% w/w.

Exemplary oral formulations of the invention include:
(a) a coxib; and
(b) a pharmaceutically acceptable carrier, wherein the composition is formulated as a solid or semi-solid dosage form.

In embodiments, the oral formulations optionally further include flavoring(s), binder(s), disintegrate(s), lubricant(s) and/or glidant(s).

Particular embodiments of formulations suitable for oral administration are as follows.

| **Oral Formulation** | | | | |
|---|---|---|---|---|
| **Ingredient** | **Ideal w/w %** | **Range w/w%** | **Purpose** | **Exemplary Alternatives** |
| Mavacoxib | 14.4 | 0.5-90 | API | any other coxib or combination thereof |
| Lactose Monohydrate (FlowLac 100) | 50.6 | 0-99.5 | flow agent, filler, diluent | siliconized MCC (sMCC), starch 1500 pre-gelatenized starch, dicalciumphosphate (Dical), calcium sulfate, Starlac and other specialized excipients |
| Microcrystalline Cellulose (Avicel PH102) | 16 | 0-99.5 | binder, disintegrant | Other binder (e.g., starch or hydroxypropylmethylcellulose (HPMC)) |
| Liver Powder | 15 | 0-40 | Flavor | Other flavors (e.g., Provestas, chicken, lamb, fish, duck, cheese, yeast, and other flavors) |
| Croscarmellose Sodium | 2.5 | 0-20 | disintegrant | Other disintegrants (e.g., sodium starch glycolate, cros-povidone, starch fully, partially and non gelatenized, MCC) |
| Stearic Acid | 0.5 | 0-50 | lubricant | magnesium stearate, sodium glycol fumerate, carbowax, PEG3350, PEG8000 |
| Colloidal Silicone Dioxide | 0.5 | 0-10 | glidant | other silicones |
| Magnesium Stearate | 0.5 | 0-50 | lubricant | magnesium stearate, sodium glycol fumerate, carbowax, PEG3350, PEG8000 |
| Other | 0 | 0-99.5 | excipients for sustained release | HPMC or other extended release excipients |
| **Total** | 100 | | | |

Particular embodiments of formulations suitable for oral administration are as follows.

| **Oral Formulation** | | | | |
|---|---|---|---|---|
| **Ingredient** | **Ideal w/w %** | **Range w/w%** | **Purpose** | **Exemplary Alternatives** |
| PAH-100 | 8 | 0.5-90 | API | any other coxib or combination thereof |
| Lactose Monohydrate (FlowLac^{™} 100) | 57 | 0-99.5 | flow agent, filler, diluent | siliconized MCC (sMCC), starch 1500 pre-gelatenized starch, dicalciumphosphate (Dical), calcium sulfate, Starlac and other specialized excipients |
| Microcrystalline Cellulose (Avicel^{™} PH102) | 16 | 0-99.5 | binder, disintegrant | Other binder (e.g., starch or hydroxypropylmethylcellulose (HPMC)) |
| Liver Powder | 15 | 0-40 | Flavor | Other flavors (e.g., Provestas, chicken, lamb, fish, duck, cheese, yeast, and other flavors) |
| Croscarmellose Sodium | 2.5 | 0-20 | disintegrant | Other disintegrants (e.g., sodium starch glycolate, cros-povidone, starch fully, partially and non gelatenized, MCC) |
| Stearic Acid | 0.5 | 0-50 | lubricant | magnesium stearate, sodium glycol fumerate, carbowax, PEG3350, PEG8000 |
| Colloidal Silicone Dioxide | 0.5 | 0-10 | glidant | other silicones |
| Magnesium Stearate | 0.5 | 0-50 | lubricant | magnesium stearate, sodium glycol fumerate, carbowax, PEG3350, PEG8000 |
| Other | 0 | 0-99.5 | excipients for sustained release | HPMC or other extended release excipients |
| **Total** | 100 | | | |

In one embodiment, an oral composition includes: i) a coxib; ii) lactose monohydrate; iii) microcrystalline cellulose; iv) flavoring; and optionally v) one or more of croscarmellose sodium, stearic acid, colloidal silicon dioxide and magnesium stearate.

In one embodiment, an oral composition includes: i) a coxib at a concentration of about 0.5 to 90% w/w; ii) lactose monohydrate at a concentration of about 1 to 99% w/w; iii) microcrystalline cellulose at a concentration of about 1 to 99% w/w; and iv) flavoring at a concentration of about 0.001 to 40% w/w.

In one embodiment, an oral composition includes: i) a coxib at a concentration of about 5 to 15% w/w; ii) lactose monohydrate at a concentration of about 50 to 60% w/w; iii) microcrystalline cellulose at a concentration of about 10 to 20% w/w; and iv) flavoring at a concentration of about 5 to 20% w/w.

In the methods described herein, an appropriate dosage level will generally be about 0.01 to about 50 mg/kg, such as, for example, 0.25 to about 25 mg/kg per day, such as 0.25 to about 20 mg/kg per day. Within this range the dosage may be 0.25 to 10, 0.5 to 6, 0.25 to 20 mg/kg, 1 to 15 mg/kg (including all intermediate dosages, such as 5.1, 5.2, 5.3 etc. mg/kg) and preferably about 1, 2, 3, 4, 5, 6, 7, 8, 9, 10, 11, 12, 13, 14 or 15 mg/kg, all in a single dosage form.

Dosing will vary by active drug, species and condition. For example, a suitable dosage level for PAH-100 is believed to be about 0.1 to 20.0 mg/kg, 0.2 to 15 mg/kg, 1 to 15 mg/kg, 3 to 10 mg/kg, 1 to 5 mg/kg and all increments thereinbetween, preferably at least 1 mg/kg or higher, such as 2, 3, 4, 5, 6, 7, 8, 9, 10, 11, 12, 13, 14, 15, 16, 17, 18, 19 or 20 mg/kg.

For example, a suitable dosage level for buprenorphine in dogs is believed to be about 0.1 to 5.0 mg/kg, 0.2 to 4.5 mg/kg, 0.3 to 4.4 mg/kg, 0.4 to 4.3 mg/kg, 0.5 to 4.2 mg/kg and all increments thereinbetween, preferably at least 0.2 mg/kg or higher, such as 0.3 mg/kg.

For the coxib compositions in particular, the compounds need only be administered by single dose, one time for an entire course of treatment to clinically resolve pain for a duration of at least about 48, 72, 96, 120, 144, or 168 hours. In this respect, "clinically resolve pain" is measured by reference to the clinically significant and measurable presence of the active in the animal's bloodstream for the requisite period of time; e.g., at least about 48, 72, 96, 120, 144, or 168 hours. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition. As such, subsequent doses may be administered as part of a therapeutic regime with administration once every 1, 2, 3, 4, 5, 6, 7 or 8 weeks. Further, different types of dosage forms may be used in combination, for example, weekly injection dosages along with weekly oral dosages.

The formulations of the invention are useful in mammals, especially companion animals, and most especially cats and dogs.

The following examples are provided to further illustrate the embodiments of the present invention, but are not intended to limit the scope of the invention. While they are typical of those that might be used, other procedures, methodologies, or techniques known to those skilled in the art may alternatively be used.

### EXAMPLE I

### Exemplary Oral Formulations

The following coxib containing formulation was prepared for oral administration.

| **Oral Formulation** | | |
|---|---|---|
| **Master Formulation** | **w/w%** | **Purpose** |
| PAH-100 | 14.4 | API |
| Lactose Monohydrate (FlowLac^{™} 100) | 50.6 | flow agent, filler, diluent |
| Microcrystalline Cellulose (Avicel^{™} PH102) | 16 | binder, disintegrant |
| Liver Powder | 15 | Flavor |
| Croscarmellose Sodium | 2.5 | disintegrant |
| Stearic Acid | 0.5 | lubricant |
| Colloidal Silicone Dioxide | 0.5 | glidant |
| Magnesium Stearate | 0.5 | lubricant |
| **Total** | 100 | |

### EXAMPLE II

### Pharmacokinetics of a Composition of the Invention

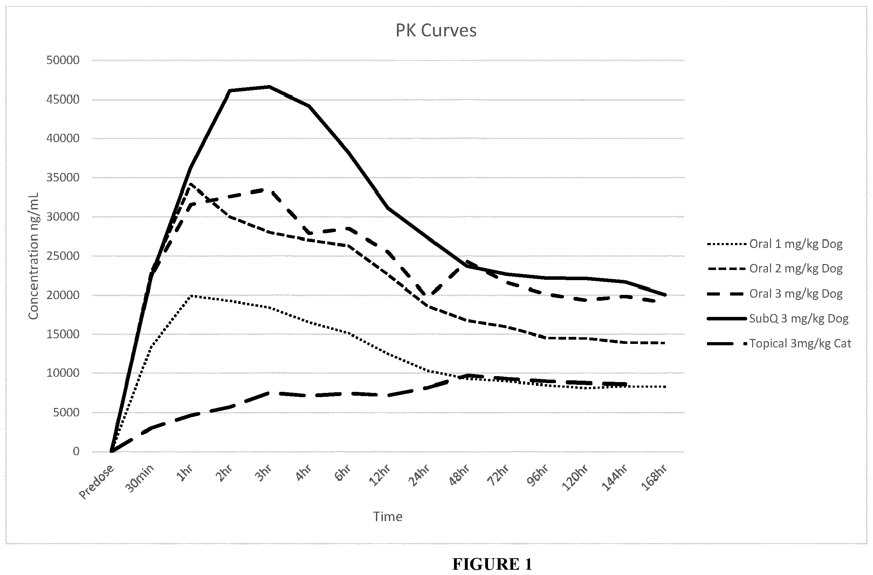

3 dogs were dosed orally with the composition of Example I at a dosing concentration of 1 mg/kg, 2 mg/kg and 3 mg/kg. Blood concentrations of coxib were present at clinically significant levels for more than 168 hours following administration of the composition, as shown in Figure 1.

### EXAMPLE III

### Exemplary Subcutaneous Injectable Formulations

The following coxib containing formulation was prepared for subcutaneous injection.

| **Injectable Formulation** | | |
|---|---|---|
| **Master Formula** | **w/w%** | **Purpose** |
| PAH-100 | 5 | API |
| Propylene Glycol | 50 | Solvent/Co-solvent |
| Polyethylene glycol 300 (PEG300) | 40 | Solvent/Co-solvent |
| Ethanol | 5 | Co-solvent, additional penetration enhancer, preservative, viscosity reducer |
| **Total** | 100 | |

### EXAMPLE IV

### Pharmacokinetics of a Composition of the Invention

1 dog was dosed via subcutaneous injection with the composition of Example III at a dosing concentration of 3 mg/kg. Blood concentrations of coxib were present at clinically significant levels for more than 168 hours following administration of the composition, as shown in Figure 1.

### EXAMPLE V

### Exemplary Topical Formulations

The following coxib containing formulation was prepared for topical administration.

| **Topical Formulation** | | |
|---|---|---|
| **Master Formula** | **w/w%** | **Purpose** |
| PAH-100 | 5 | API |
| Propylene Glycol (PG) | 40 | Solvent |
| Propylene Carbonate (PC) | 40 | Penetration Enhancer |
| Ethanol | 15 | Co-solvent, additional penetration enhancer, preservative, viscosity reducer |
| **Total** | 100 | |

### Example VI

### Pharmacokinetics of a Composition of the Invention

1 cat was dosed topically with the composition of Example V at a dosing concentration of 3 mg/kg. Blood concentrations of coxib were present at clinically significant levels for more than 168 hours following administration of the composition, as shown in Figure 1.

### Example VII

### Exemplary Topical Formulation

The following coxib containing formulation was prepared for topical administration.

| **Topical Formulation** | | |
|---|---|---|
| **Master Formula** | **w/w%** | **ID: PAH-16-04-004** |
| PAH-100 | 7.5 | |
| Propylene Glycol (PG) | 45 | |
| Propylene Carbonate (PC) | 27.5 | |
| n-methyl pyrrolidone (NMP) | 20 | |
| **Total** | 100 | |

### Example VIII

### Exemplary Oral Formulations

The following coxib containing formulation was prepared for oral administration.

| **Oral Formulation** | | |
|---|---|---|
| **Master Formulation** | **w/w%** | **Purpose** |
| PAH-100 racemic mixture (D90 - 14.7µm)* | 8.00% (R+S) (results in 4.00% S Label Claim) | API |
| Lactose Monohydrate, NF (Flowlac^{™} 100 or #316 Fast Flo^{™})** | 57 | flow agent, filler, diluent |
| Microcrystalline Cellulose (Avicel^{™} PH102) | 16 | binder, disintegrant |
| Liver Powder | 15 | Flavor |
| Croscarmellose Sodium | 2.5 | disintegrant |
| Stearic Acid | 0.5 | lubricant |
| Colloidal Silicone Dioxide | 0.5 | glidant |
| Magnesium Stearate | 0.5 | lubricant |
| **Total** | 100 | |
| * API quantity is adjusted for purity and water content. | | |
| ** Excipient quantity is adjusted as needed. | | |
| **Tablet weights of 250, 375, 1000 and 1500 mg yield tablet strengths of 10, 15, 40 and 60 mg respectively. Each tablet had a bisect.** | | |

### Compounding Procedures

1. Weigh out all ingredients.
2. Screen (20 mesh) all ingredients in the following order: 1/2 Lactose Monohydrate, 1/2 Microcrystalline Cellulose, remaining excipients, PAH-100 API, ending with 1/2 Microcrystalline Cellulose and 1/2 Lactose Monohydrate.

Note: For scale up, consider holding back the Stearic Acid and Magnesium Stearate and dividing the blend time where 1/2 of the pre-blend is removed Stearic Acid and Magnesium Stearate are sieved into the blender and the 1/2 pre-blend is added back into the blender.

Run V-blender for 300 seconds (approximately 300 revolutions).

### Tableting Procedure

1. Set up tablet press for 250, 375, 1000, or 1500 mg tablets. All tooling will result in a scored tablet.
2. Check tablet weights and hardness during start up.
3. Check tablet weights periodically throughout batch and, if needed, adjust tablet weight and hardness.
4. Package the bulk tablets and apply a label to the container(s).

### EXAMPLE IX

### Exemplary Subcutaneous Injectable Formulations

The following coxib containing formulation was prepared for subcutaneous injection.

| **Injectable Formulation** | | |
|---|---|---|
| **Master Formula** | **w/w%** | **Purpose** |
| PAH-100* (delivered as 50/50 racemic mixture) | 12.00% (R+S) (results in 6.00% S Label Claim) | API |
| Propylene Glycol | 50 | Solvent/Co-solvent |
| Polyethylene glycol 300 (PEG300)** | 33 | Solvent/Co-solvent |
| Ethanol | 5 | Co-solvent, additional penetration enhancer, preservative, viscosity reducer |
| **Total** | 100 | |
| * API quantity is adjusted for purity and water content. | | |
| ** Excipient quantity is adjusted based upon API correction factor. | | |

### Compounding Procedures

1. Obtain tare weight of formulation vessel.
2. Weigh out and add Propylene Glycol, PEG 300, and Ethanol to formulation vessel.
3. Begin mixing and wait for visual homogeneity.
4. Add PAH-100 to formulation vessel.
5. Mix until visual dissolution at room temperature.

Note: For a 300g product batch, using micronized PAH-100 (d90=14.55m), dissolution was achieved after approx. 1-1.5 hours (batch 20180515M-5). With non-micronized material (d90=88.17m), dissolution was achieved after approx. 2-2 'A hours (batch 20180412M-82).
6. Check batch weight to account for evaporated Ethanol. If needed, add Ethanol to achieve target Ethanol quantity.
7. Turn off mixer.
8. Sterile filter product and aseptically fill into vials.

### EXAMPLE X

### Exemplary Topical Formulations

The following coxib containing formulation was prepared for topical administration.

| **Topical Formulation** | | |
|---|---|---|
| **Master Formula** | **w/w%** | **Purpose** |
| PAH-100* (delivered as 50/50 racemic mixture) | 5 | API |
| Propylene Glycol (PG) | 40 | Solvent |
| Propylene Carbonate (PC) | 40 | Penetration Enhancer |
| Ethanol * * | 15 | Co-solvent, additional penetration enhancer, preservative, viscosity reducer |
| **Total** | 100 | |
| * API quantity is adjusted purity and moisture. | | |
| ** Excipient quantity is adjusted as needed. | | |

### Compounding Procedures

1. Weight out and add Propylene Glycol, Propylene Carbonate, and Ethanol to formulation vessel.
2. Begin mixing and wait for visual homogeneity.
3. Add PAH-100 to formulation vessel.
4. Mix until visual dissolution at room temperature.
5. Turn off mixer.
6. Filter through a Whatman 1 paper filter (or similar, for lab scale).

### EXAMPLE XI

### Safety And Pharmacokinetics of a Composition of the Invention

### 6% (S) PAH-100 Injection for Dogs

Safety and pharmacokinetic study PAH18-21 evaluated a single subcutaneous injection of a 6% PAH-100 formulation (formulation of Example IX) in eight dogs at a dosing concentration of 5 mg/kg. Injection site reactions consisted of barely perceptible to well defined erythema and edema and scabbing. Clinical observations included soft feces 5 - 11 days post dosing. One dog developed a salivary mucocele at the blood collection site. This reaction is not related to blood collection or treatment with a 6% PAH-100 injectable formulation.

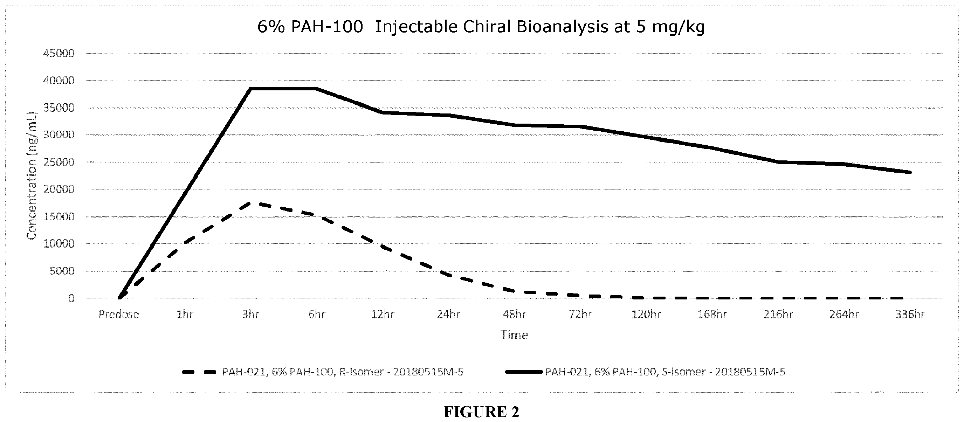

Figure 2: Mean 6% PAH-100 Plasma Concentration-Time Profiles of the (R) and (S) Isomers Following a Single Subcutaneous Injection of 5 mg/kg in Dogs. Figure 2 illustrates the chiral analysis of the (R) and (S) isomer in the plasma of dogs dosed at 5 mg/kg with a 6% PAH-100 injectable formulation prepared as described in Example IX.

### EXAMPLE XII

### Safety And Pharmacokinetics of a Composition of the Invention

### 5% PAH-100 Injectable for Dogs

Two safety/ pharmacokinetic studies, (PAH17-015 and PAH18-011) were conducted at 3 mg/kg with a single injection of a 5% PAH-100 formulation (formulation of Example IX) in dogs. In both studies, dogs were dosed subcutaneously between the shoulder blades and injection site reactions were evaluated. Dermal irritation at the site of injection consisted of discoloration and scabbing, warm to the touch, and slight erythema. Clinical observations included vocalization, salivation, excessive scratching at the injection site and soft feces.

Study PAH17-024 evaluated a once a week subcutaneous dose of a 5% PAH-100 formulation (formulation of Example IX) at dosing concentrations of 3 mg/kg and 15 mg/kg for 3-weeks. Clinical observations and dermal irritation at the site of injection were identical to dogs administered a single 3 mg/kg dose. Additional safety assessments included in this study was based on electrocardiographic examinations, clinical and anatomic pathology. Results confirmed there was no effect of the subcutaneous injection of PAH-100 on qualitative or quantitative ECG parameters, clinical pathology values, and histologic findings. Red discoloration in the subcutis at the injection site of 1 dog dosed at 3 mg/kg dose. All other macroscopic changes were considered incidental and not related to the test article.

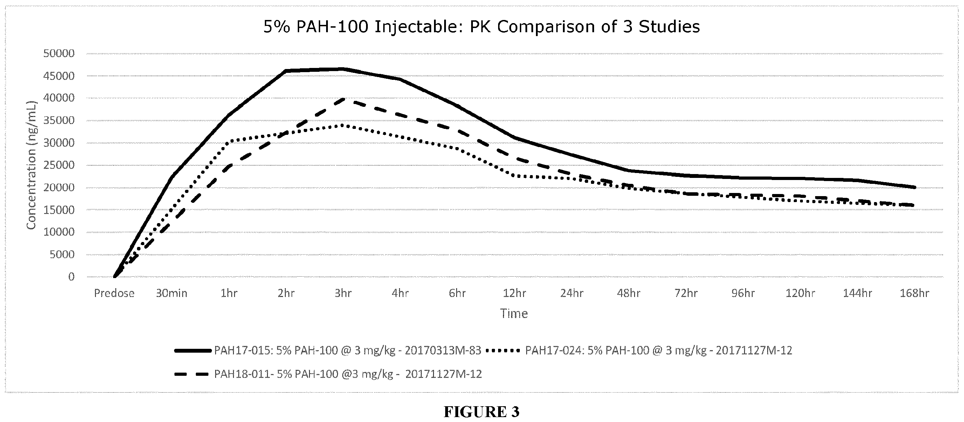

Figure 3: Mean 5% PAH-100 Plasma Concentration-Time Profiles Following a Single Subcutaneous Injection of 3 mg/kg in Dogs. Figure 3 illustrates the PK curves of the first 3 mg/kg injection from all three studies.

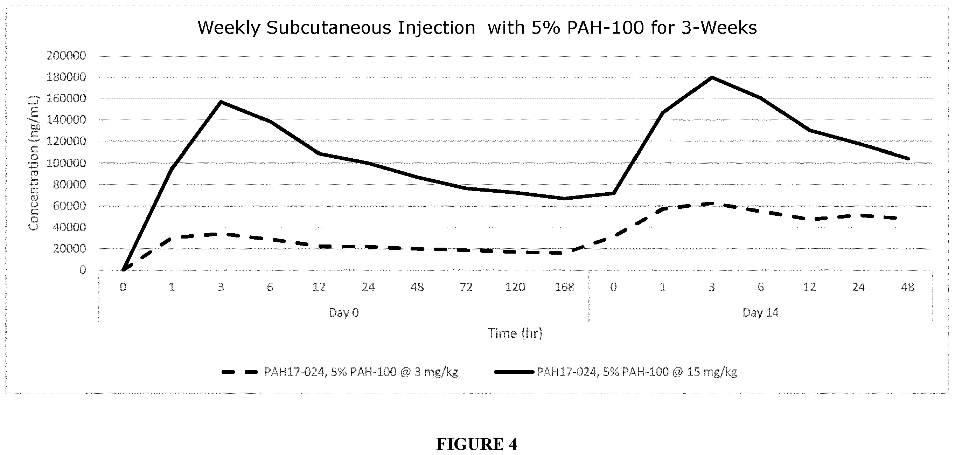

Figure 4: Mean Plasma Concentration-Time Profiles Following Weekly Subcutaneous Injection for 3-Weeks. Figure 4 details the plasma concentrations in dogs dosed at 3 mg/kg and 15 mg/kg weekly for 3-weeks, Study PAH17-024.

### EXAMPLE XIII

### Safety And Pharmacokinetics of a Composition of the Invention

### 5% PAH-100 Injectable and Tablet Treatment in Dogs

Study PAH17-016 assessed the safety and pharmacokinetics of a dosing regimen where dogs were received a single subcutaneous injection of a 5% PAH-100 formulation (formulation of Example IX) at dosing concentrations of 2 mg/kg or 10 mg/kg, followed by a weekly oral administration with PAH-100 tablets (formulation of Example VIII) starting 7 days post-injection. Dogs were orally dosed once a week for 6 weeks at dosing concentrations of 1 mg/kg or 5 mg/kg. Injection site reactions, clinical pathology values, and histologic findings were evaluated. Dermal irritation and clinical observations included excessive scratching, mild hair loss, and scabbing at injection site, vocalization during injection, salivation, and soft feces. A nodule developed at the injection site of one dog. There were no test article effects on clinical pathology endpoints. There was no test article related changes macroscopically or microscopically in tissues collected at necropsy.

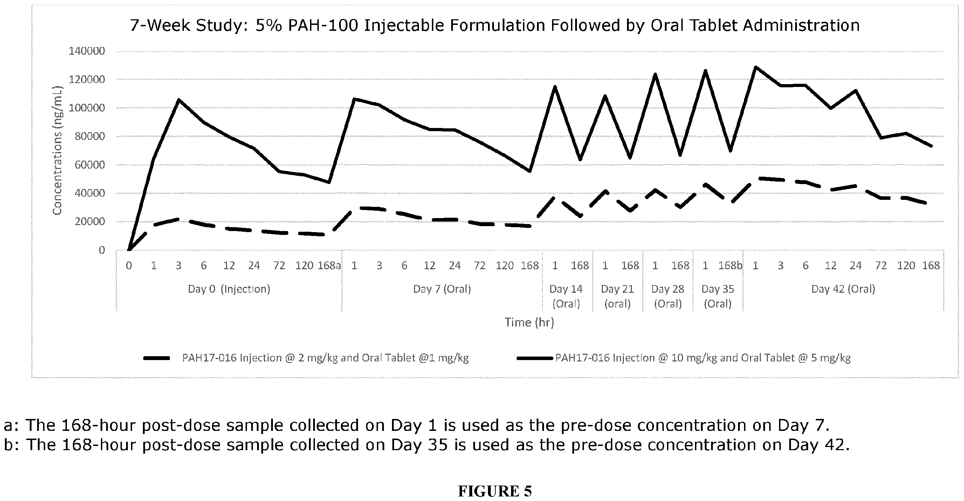

Figure 5: Mean Plasma Concentration-Time Profiles Following a Single Subcutaneous Injection Followed by Oral Tablet Administration at 1X and 5X dose. Figure 5 illustrates the plasma concentrations in dogs subcutaneously treated weekly for 3-weeks at 3 mg/kg and 15 mg/kg.

### EXAMPLE XIV

### Safety And Pharmacokinetics of a Composition of the Invention

### PAH-100 Oral Tablet Efficacy (PAH16-013)

Nine purpose bred hound dogs with naturally occurring coxofemoral joint disease received each of three treatments, a single oral dose of PAH-100 (3 mg/kg; formulation of Example VIII), 2) no treatment, or 3) carprofen (4.4 mg/kg, PO, q24 hr, 7 doses total). Efficacy was determined by rear limb using a quantified kinetic gait analysis. Blood samples were collected to determine PAH-100 plasma concentration levels. No abnormal clinical observations or adverse events occurred in any of the dogs. Confirming a single dose of PAH-100 at 3 mg/kg is well tolerated. Efficacy results indicate improved limb use in dogs with coxofemoral joint osteoarthritis over a period of 7 days after oral administration of PAH 100 compared to no treatment and daily carprofen administration based on a majority of kinetic outcomes.

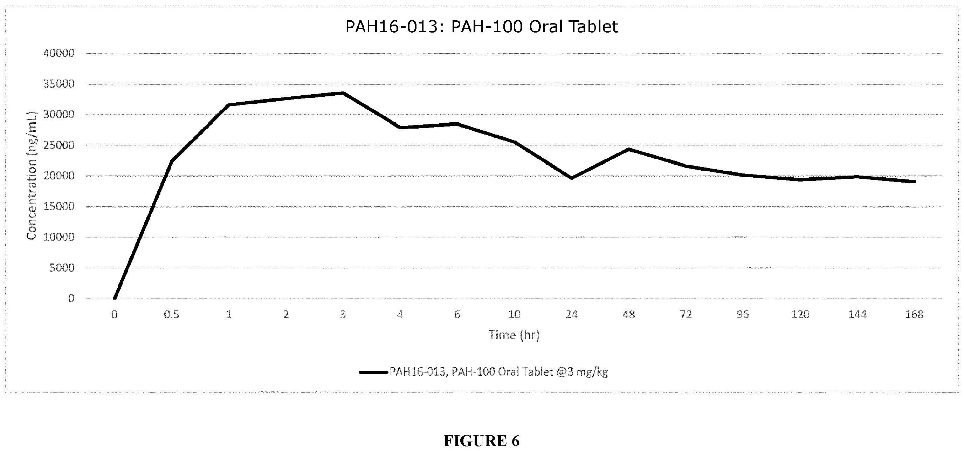

Figure 6: Mean Plasma Concentration-Time Profiles Following Oral Administration with PAH-100 Tablet at 3 mg/kg. Figure 6 details the mean PAH-100 plasma concentrations in dogs treated orally at 3 mg/kg.

### EXAMPLE XV

### Field Safety and Efficacy for Postoperative Pain

This study evaluated the field safety and effectiveness of a single subcutaneous injection of PAH-100 (formulation of Example IX) for control of postoperative pain associated with either ovariohysterectomy (OVH) or cranial cruciate ligament (CCL) repair in dogs. The investigational veterinary product (IVP; formulation of Example IX) dose rate was 3 mg/kg, but Protocol Amendment 1 raised the dose to 5 mg/kg after 24 dogs were enrolled. Forty-nine (49) dogs were enrolled at 2 study sites. Thirteen (13) dogs were treated with the IVP at a one-time dose rate of 5 mg/kg, 12 dogs were treated with the IVP at a one-time dose rate of 3 mg/kg, and 24 dogs were treated with the CVP (saline). All 25 dogs treated with IVP were included in the safety evaluation, while 12 dogs treated at the 5 mg/kg dose rate and 24 dogs treated with CVP were included in the primary efficacy evaluation. Dogs receiving the 3 mg/kg IVP dose are considered supplemental cases.

Dogs enrolled in the study were undergoing either CCL extracapsular repair or OVH surgery. On Day 0, a physical examination was conducted, a baseline pain assessment score was assigned, and blood and serum samples were collected for clinical pathology testing. The IVP or CVP was given 1 hour prior to intubation and then surgery was performed. Dogs were assigned pain scores at specified times through 8 hours post-extubation, unless they required rescue analgesia for pain scores of 6 or greater.

On Day 1, the injection site was evaluated for all dogs, including ones that had received rescue analgesia. Pain assessments continued through 30 hours post-extubation unless rescue analgesia was required.

On Day 2, the injection site was evaluated for all dogs, including ones that had received rescue analgesia. Pain assessments continued through 54 hours post-extubation unless rescue analgesia was required.

On Day 3, a physical examination and injection site evaluation was conducted, and blood and serum was collected for clinical pathology testing. For dogs that had not received rescue analgesia, a final pain assessment was conducted at 72 hours post-extubation.

Based on success being defined as pain scores < 6 through the 48-hour assessment, the primary IVP efficacy in the 6 OVH cases and 6 CCL cases was 33.3% and 83.3%, respectively. The CVP efficacy in the 13 OVH cases and 11 CCL cases was 38.5% and 45.5%, respectively.

There were no serious adverse events and only one case of injection discomfort was judged to be related to IVP administration, while one case of diarrhea was judged to be possibly related to IVP administration. Thus, the low frequency of adverse events attributable to IVP administration demonstrated safe use of this formulation.

Although the objects of the disclosure have been described with reference to the above example, it will be understood that modifications and variations are encompassed within the spirit and scope of the disclosure. Accordingly, the disclosure is limited only by the claims. Aspects and features of the present disclosure are set out in the following numbered clauses which contain the subject matter of the claims of the parent application.
**1.** An injectable pharmaceutical composition, comprising:
   a) a cyclooxygenase-2 (COX-2) inhibitor, wherein the COX-2 inhibitor is mavacoxib, a compound of Formula (I): 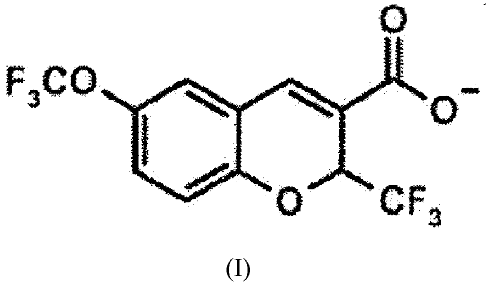 or an isomer or pharmaceutically acceptable salt thereof, a compound of Formula (II): 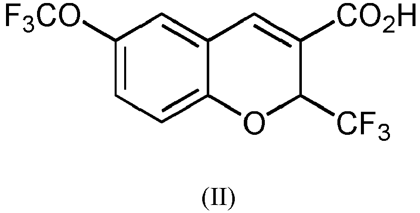 or an isomer or pharmaceutically acceptable salt thereof, or any combination thereof; and
   b) a solvent, wherein the composition is formulated for subcutaneous administration.
**2.** The composition of clause 1, wherein the COX-2 inhibitor is selected from the group consisting of: Tris(hydroxymethyl)aminomethane (±)-6-(trifluoromethoxy)-2-(trifluoromethyl)-2H-chromene-3-carboxylate, (±)-6-(trifluoromethoxy)-2-(trifluoromethyl)-2H-chromene-3-carboxylic acid, Tris(hydroxymethyl)aminomethane (R)-6-(trifluoromethoxy)-2-(trifluoromethyl)-2H-chromene-3-carboxylate, (R)-6-(trifluoromethoxy)-2-(trifluoromethyl)-2H-chromene-3-carboxylic acid, Tris(hydroxymethyl)aminomethane (S)-6-(trifluoromethoxy)-2-(trifluoromethyl)-2H-chromene-3-carboxylate, (S)-6-(trifluoromethoxy)-2-(trifluoromethyl)-2H-chromene-3-carboxylic acid, and any combination thereof.
**3.** The composition of clause 1, wherein the COX-2 inhibitor is a compound of Formula (III): 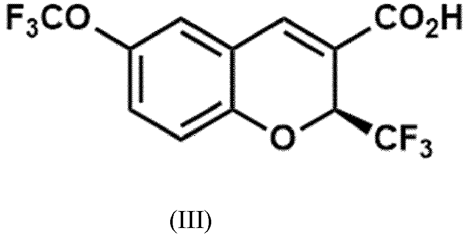 or an isomer or pharmaceutically acceptable salt thereof.
**4.** The composition of clause 1, wherein the COX-2 inhibitor is mavacoxib.
**5.** The composition of clause 1, wherein the composition is non-aqueous.
**6.** The composition of clause 1, further comprising propylene glycol present at over 1% w/w of the composition.
**7.** The composition of clause 6, wherein the propylene glycol is present at about 60% w/w or less of the composition.
**8.** The composition of clause 6, wherein the propylene glycol is present at about 50% w/w.
**9.** The composition of clause 1, further comprising polyethylene glycol present at about 85% w/w or less of the composition.
**10.** The composition of clause 9, wherein the polyethylene glycol is present at about 30 to 35% w/w or less of the composition.
**11.** The composition of clause 1, further comprising ethanol present at about 25% w/w or less of the composition.
**12.** The composition of clause 1, wherein the COX-2 inhibitor is present at about 5, 6, 7, 8, 9, 10, 11, 12, 13, 14, or 15% w/w of the composition.
**13.** The composition of clause 1, further comprising an anti-oxidant.
**14.** The composition of clause 13, wherein the anti-oxidant is present at no more than about 10% w/w of the composition.
**15.** The composition of clause 1, wherein the composition comprises:
   i) COX-2 inhibitor;
   ii) propylene glycol;
   iii) polyethylene glycol;
   iv) ethanol; and optionally
   v) an anti-oxidant.
**16.** The composition of clause 1, wherein the composition comprises:
   i) COX-2 inhibitor at a concentration of about 0.5 to 50% w/w;
   ii) propylene glycol at a concentration of about 1 to 60% w/w;
   iii) polyethylene glycol at a concentration of about 0.5 to 85% w/w; and
   iv) ethanol at a concentration of about 0.001 to 25% w/w.
**17.** The composition of clause 1, wherein the composition comprises:
   i) COX-2 inhibitor at a concentration of about 5 to 15% w/w;
   ii) propylene glycol at a concentration of about 45 to 55% w/w;
   iii) polyethylene glycol at a concentration of about 30 to 35% w/w; and
   iv) ethanol at a concentration of about 1 to 10% w/w.
**18.** The composition of clause 1, wherein at least about 5,000, 10,000, 15,000 or 20,000 ng/ml of the COX-2 inhibitor is present in the blood stream of the subject for at least about 48, 60, 72, 84, 96, 108, 120, 132, 144, 156, 168 hours or greater upon administration to a mammal.
**19.** The composition of clause 1, further comprising buprenorphine.
**20.** The composition of clause 19, wherein buprenorphine is present in a dose of about 0.1, 0.2, 0.3, 0.4, 0.5, 1.0, 1.5, 2.0, 2.5, 3.0, 3.5, 4.0, 4.5, 5.0, 5.5, 6.0, 6.5 or 7.0 mg/kg.
**21.** The composition of clause 1, wherein the composition is stable at room temperature for at least 6 months.
**22.** The composition of clause 21, wherein the composition is stable at room temperature for at least 12 months.
**23.** A pharmaceutical composition for topical administration, comprising:
   a) a cyclooxygenase-2 (COX-2) inhibitor, wherein the COX-2 inhibitor is mavacoxib, a compound of Formula (I): 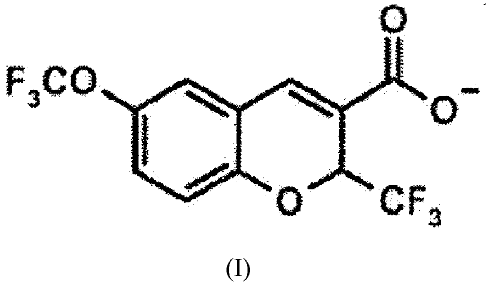 or an isomer or pharmaceutically acceptable salt thereof, a compound of Formula (II): 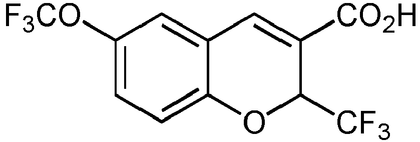 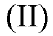 or an isomer or pharmaceutically acceptable salt thereof, or any combination thereof; and
   b) a solvent; wherein the composition is formulated as a topical dosage form.
**24.** The composition of clause 23, wherein the COX-2 inhibitor is selected from the group consisting of: Tris(hydroxymethyl)aminomethane (±)-6-(trifluoromethoxy)-2-(trifluoromethyl)-2H-chromene-3-carboxylate, (±)-6-(trifluoromethoxy)-2-(trifluoromethyl)-2H-chromene-3-carboxylic acid, Tris(hydroxymethyl)aminomethane (R)-6-(trifluoromethoxy)-2-(trifluoromethyl)-2H-chromene-3-carboxylate, (R)-6-(trifluoromethoxy)-2-(trifluoromethyl)-2H-chromene-3-carboxylic acid, Tris(hydroxymethyl)aminomethane (S)-6-(trifluoromethoxy)-2-(trifluoromethyl)-2H-chromene-3-carboxylate, (S)-6-(trifluoromethoxy)-2-(trifluoromethyl)-2H-chromene-3-carboxylic acid, and any combination thereof.
**25.** The composition of clause 23, wherein the COX-2 inhibitor is a compound of Formula (III): 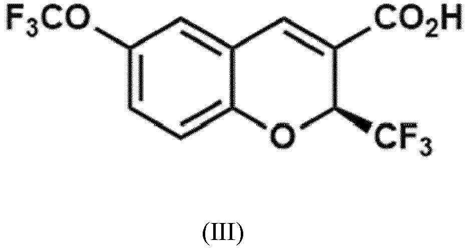 or an isomer or pharmaceutically acceptable salt thereof.
**26.** The composition of clause 23, wherein the COX-2 inhibitor is mavacoxib.
**27.** The composition of clause 23, wherein the composition is non-aqueous.
**28.** The composition of clause 23, further comprising propylene glycol present at over 1% w/w of the composition.
**29.** The composition of clause 28, wherein the propylene glycol is present at about 99% w/w or less of the composition.
**30.** The composition of clause 28, wherein the propylene glycol is present at about 40% w/w or less of the composition.
**31.** The composition of clause 23, further comprising propylene carbonate present at about 75% w/w or less of the composition.
**32.** The composition of clause 31, wherein the propylene carbonate is present at about 40% w/w.
**33.** The composition of clause 23, further comprising ethanol present at about 25% w/w or less of the composition.
**34.** The composition of clause 23, wherein the COX-2 inhibitor is present at about 5, 6, 7, 8, 9, 10, 11, 12, 13, 14, or 15% w/w of the composition.
**35.** The composition of clause 23, further comprising an anti-oxidant.
**36.** The composition of clause 35, wherein the anti-oxidant is present at no more than about 10% w/w of the composition.
**37.** The composition of clause 23, wherein the composition comprises:
   i) COX-2 inhibitor;
   ii) propylene glycol;
   iii) propylene carbonate;
   iv) ethanol; and optionally
   v) an anti-oxidant.
**38.** The composition of clause 23, wherein the composition comprises:
   i) COX-2 inhibitor at a concentration of about 0.5 to 50% w/w;
   ii) propylene glycol at a concentration of about 1 to 99% w/w;
   iii) propylene carbonate at a concentration of about 0.001 to 75% w/w; and
   iv) ethanol at a concentration of about 0.001 to 25% w/w.
**39.** The composition of clause 23, wherein the composition comprises:
   i) COX-2 inhibitor at a concentration of about 5 to 15% w/w;
   ii) propylene glycol at a concentration of about 35 to 45% w/w;
   iii) propylene carbonate at a concentration of about 35 to 45% w/w; and
   iv) ethanol at a concentration of about 5 to 20% w/w.
**40.** The composition of clause 23, wherein at least about 5,000, 10,000, 15,000 or 20,000 ng/ml of the COX-2 inhibitor is present in the blood stream of the subject for at least about 48, 60, 72, 84, 96, 108, 120, 132, 144, 156, 168 hours or greater upon administration to a mammal.
**41.** The composition of clause 23, further comprising buprenorphine.
**42.** The composition of clause 41, wherein buprenorphine is present in a dose of about 0.1, 0.2, 0.3, 0.4, 0.5, 1.0, 1.5, 2.0, 2.5, 3.0, 3.5, 4.0, 4.5, 5.0, 5.5, 6.0, 6.5 or 7.0 mg/kg.
**43.** The composition of clause 23, wherein the composition is stable at room temperature for at least 6 months.
**44.** The composition of clause 43, wherein the composition is stable at room temperature for at least 12 months.
**45.** A pharmaceutical composition for oral administration, comprising:
   a) a cyclooxygenase-2 (COX-2) inhibitor, wherein the COX-2 inhibitor is mavacoxib, a compound of Formula (I): 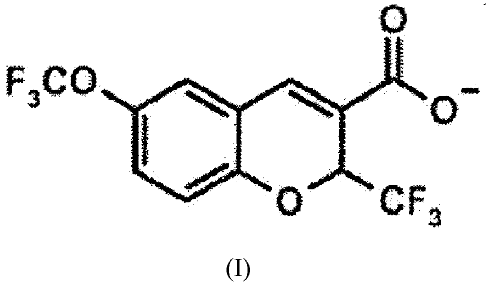 or an isomer or pharmaceutically acceptable salt thereof, a compound of Formula (II): 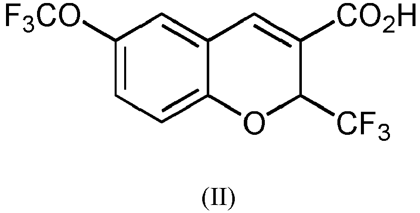 or an isomer or pharmaceutically acceptable salt thereof, or any combination thereof; and
   b) a pharmaceutically acceptable carrier, wherein the composition is formulated as a solid or semi-solid oral dosage form.
**46.** The composition of clause 45, wherein the COX-2 inhibitor is selected from the group consisting of: Tris(hydroxymethyl)aminomethane (±)-6-(trifluoromethoxy)-2-(trifluoromethyl)-2H-chromene-3-carboxylate, (±)-6-(trifluoromethoxy)-2-(trifluoromethyl)-2H-chromene-3-carboxylic acid, Tris(hydroxymethyl)aminomethane (R)-6-(trifluoromethoxy)-2-(trifluoromethyl)-2H-chromene-3-carboxylate, (R)-6-(trifluoromethoxy)-2-(trifluoromethyl)-2H-chromene-3-carboxylic acid, Tris(hydroxymethyl)aminomethane (S)-6-(trifluoromethoxy)-2-(trifluoromethyl)-2H-chromene-3-carboxylate, (S)-6-(trifluoromethoxy)-2-(trifluoromethyl)-2H-chromene-3-carboxylic acid, and any combination thereof.
**47.** The composition of clause 45, wherein the COX-2 inhibitor is a compound of Formula (III): 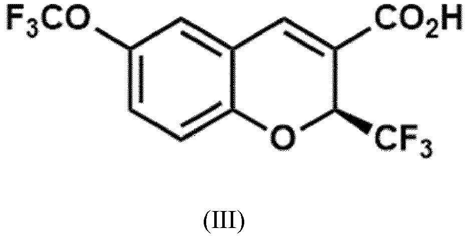 or an isomer or pharmaceutically acceptable salt thereof.
**48.** The composition of clause 45, wherein the COX-2 inhibitor is mavacoxib.
**49.** The composition of clause 45, further comprising lactose monohydrate present at over 1% w/w of the composition.
**50.** The composition of clause 49, wherein the lactose monohydrate is present at about 99% w/w or less of the composition.
**51.** The composition of clause 49, wherein the lactose monohydrate is present at about 50% w/w or less of the composition.
**52.** The composition of clause 45, further comprising microcrystalline cellulose present at about 99% w/w or less of the composition.
**53.** The composition of clause 31, wherein the microcrystalline cellulose is present at about 15 to 20% w/w.
**54.** The composition of clause 45, further comprising flavoring, croscaremellose sodium, stearic acid, colloidal silicon dioxide, magnesium stearate, or any combination thereof.
**55.** The composition of clause 45, wherein the COX-2 inhibitor is present at about 5, 6, 7, 8, 9, 10, 11, 12, 13, 14, or 15% w/w of the composition.
**56.** The composition of clause 45, wherein the composition comprises:
   i) COX-2 inhibitor;
   ii) lactose monohydrate;
   iii) microcrystalline cellulose;
   iv) flavoring; and optionally
   v) one or more of croscarmellose sodium, stearic acid, colloidal silicon dioxide and magnesium stearate.
**57.** The composition of clause 45, wherein the composition comprises:
   i) COX-2 inhibitor at a concentration of about 0.5 to 90% w/w;
   ii) lactose monohydrate at a concentration of about 1 to 99% w/w;
   iii) microcrystalline cellulose at a concentration of about 1 to 99% w/w; and
   iv) flavoring at a concentration of about 0.001 to 40% w/w.
**58.** The composition of clause 45, wherein the composition comprises:
   i) COX-2 inhibitor at a concentration of about 5 to 15% w/w;
   ii) lactose monohydrate at a concentration of about 50 to 60% w/w;
   iii) microcrystalline cellulose at a concentration of about 10 to 20% w/w; and
   iv) flavoring at a concentration of about 5 to 20% w/w.
**59.** The composition of clause 45, wherein at least about 5,000, 10,000, 15,000 or 20,000 ng/ml of the COX-2 inhibitor is present in the blood stream of the subject for at least about 48, 60, 72, 84, 96, 108, 120, 132, 144, 156, 168 hours or greater upon administration to a mammal.
**60.** The composition of clause 45, further comprising buprenorphine.
**61.** The composition of clause 60, wherein buprenorphine is present in a dose of about 0.1, 0.2, 0.3, 0.4, 0.5, 1.0, 1.5, 2.0, 2.5, 3.0, 3.5, 4.0, 4.5, 5.0, 5.5, 6.0, 6.5 or 7.0 mg/kg.
**62.** A method of treating a disease or disorder in a subject, comprising administering to the subject an effective amount of a composition of any of clauses 1-61.
**63.** The method of clause 62, wherein the subject is a mammal.
**64.** The method of clause 63, wherein the subject is a canine.
**65.** The method of clause 63, wherein the subject is a feline.
**66.** The method of clause 63, wherein the disease or disorder is pain or inflammation.
**67.** The method of clause 62, wherein the disease or disorder is an inflammatory disease.
**68.** The composition of clause 62, wherein at least about 5,000, 10,000, 15,000 or 20,000 ng/ml of the COX-2 inhibitor is present in the blood stream of the subject for at least about 48, 60, 72, 84, 96, 108, 120, 132, 144, 156, 168 hours or greater upon administration to a mammal.
**69.** The method of clause 62, comprising administering the injectable pharmaceutical composition followed by administration of the oral pharmaceutical composition or the topical pharmaceutical composition.
**70.** The method of clause 69, wherein the injectable pharmaceutical composition is administered weekly and the oral pharmaceutical composition or the topical pharmaceutical composition is administered weekly.

## Claims

1. A pharmaceutical composition for oral administration, comprising:
a) a cyclooxygenase-2 (COX-2) inhibitor, wherein the COX-2 inhibitor is mavacoxib, a compound of Formula (II): 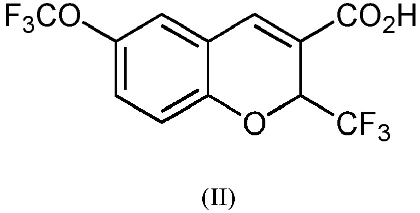 or a pharmaceutically acceptable salt thereof, or any combination thereof; and
b) a pharmaceutically acceptable carrier, wherein the composition is formulated as a liquid suspension, a solid or a semi-solid oral dosage form.

2. The composition of claim 1, wherein the pharmaceutically acceptable salt of the COX-2 inhibitor is Tris(hydroxymethyl)aminomethane (±)-6-(trifluoromethoxy)-2-(trifluoromethyl)-2H-chromene-3-carboxylate, and optionally wherein the pharmaceutically acceptable salt of the COX-2 inhibitor is micronized.

3. The composition of claim 1, wherein at least about 5,000, 10,000, 15,000 or 20,000 ng/ml of the COX-2 inhibitor is present in the blood stream of a subject for at least about 48, 60, 72, 84, 96, 108, 120, 132, 144, 156, 168 hours or greater after administration to a subject.

4. The composition of claim 1, further comprising a glidant.

5. The composition of claim 4, wherein the glidant is colloidal silicon dioxide present from about 0 to 10% w/w of the composition.

6. The composition of claim 1, further comprising a disintegrant.

7. The composition of claim 6, wherein the disintegrant is sodium starch glycolate present from about 0 to 20% w/w of the composition.

8. The composition of claim 1, further comprising a lubricant.

9. The composition of claim 8, wherein the lubricant is a stearic acid lubricant present from about 0 to 50% w/w of the composition.

10. The composition of claim 1, wherein the composition is a single oral dose comprising a weight of about 250, 375, 100 or 1500 mg,
wherein the COX-2 inhibitor comprises about 10, 15, 40 or 60 mg in the composition.

11. The composition of claim 1, wherein:
i) the COX-2 inhibitor is present at a concentration of 0.5 to 50% w/w;
ii) a glidant is present at a concentration of 0 to 10% w/w;
iii) a disintegrant is present at a concentration of 0 to 20% w/w; and
iv) a lubricant is present at a concentration of 0 to 50% w/w.

12. The composition as defined in any preceding claims for use as a medicament.

13. The composition as defined in any one of claims 1 to 11 for use in a method of treating pain or inflammation, or an inflammatory disease in a subject, optionally wherein the subject is a mammal, optionally wherein the mammal is a canine or a feline.

14. The composition for use according to claim 13, wherein a therapeutically effective amount of the composition is administered in a single dose.

15. The composition for use according to claim 13, wherein
i) at least about 5,000, 10,000, 15,000 or 20,000 ng/ml of the COX-2 inhibitor is present in the blood stream of the subject for at least about 48, 60, 72, 84, 96, 108, 120, 132, 144, 156, 168 hours or greater upon administration to a mammal; and/or
ii) the use comprises administering an injectable pharmaceutical composition followed by administration of the oral pharmaceutical composition or a topical pharmaceutical composition, wherein the injectable pharmaceutical composition comprises:
a) a cyclooxygenase-2 (COX-2) inhibitor, wherein the COX-2 inhibitor is mavacoxib, a compound of Formula (I): 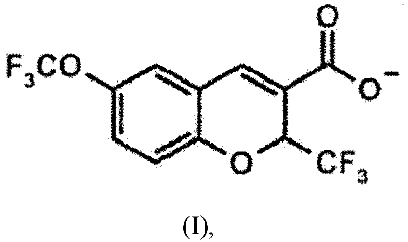 or a pharmaceutically acceptable salt thereof, a compound of Formula (II): 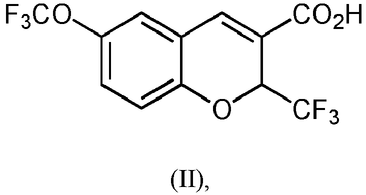 or a pharmaceutically acceptable salt thereof, or any combination thereof; and
b) propylene glycol;
c) polyethylene glycol; and
d) ethanol;
wherein the topical pharmaceutical composition comprises:
a) a cyclooxygenase-2 (COX-2) inhibitor, wherein the COX-2 inhibitor is mavacoxib, a compound of Formula (I): 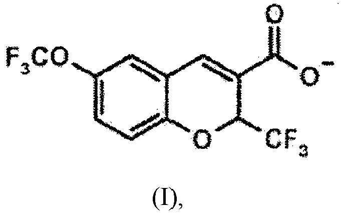 or a pharmaceutically acceptable salt thereof, a compound of Formula (II): 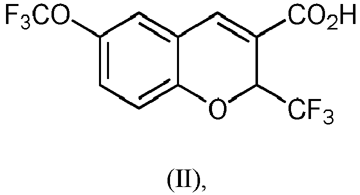 or a pharmaceutically acceptable salt thereof, or any combination thereof; and
b) a solvent; wherein the composition is formulated as a topical dosage form; optionally wherein the injectable pharmaceutical composition is administered weekly and the oral pharmaceutical composition or the topical pharmaceutical composition is administered weekly.
